# EUROPEAN PATENT APPLICATION

(11) **EP 2 153 893 A1**
(43) Date of publication of application: **17.02.2010**
(21) Application number: 08160902.6
(22) Date of filing: 22.07.2008
(51) Int. Cl.: B01J 21/06, B01J 27/02, B01J 37/00, B01J 37/20

(54) **Sulfated zirconia catalyst; its production by melting the precursors and its use for esterification of fatty acids with alcohols.**

(71) Applicant: Yellow Diesel B.V., 1018 WB Amsterdam (NL)
(72) Inventor: Rothenberg, Gadi, 1096 HS, Amsterdam (NL); Dimian, Alexandre C., 1183 EC, Amstelveen (NL)
(74) Representative: van Looijengoed, Ferry Antoin Theodorus

(57) **Abstract**

The present invention pertains to a solid acid catalyst of the formula M1ₐM2_{b}Oₓ(AO3H)y, wherein M1 and M2 may be the same or different and wherein M1 is selected from one or more of the transition metals, indium, and tin and M2 is selected from one or more of the transition metals, the lanthanide metals, indium, and tin, A is selected from sulphur, phosphorus, and mixtures thereof, a is in the range of 0.01 to 1, b is in the range of 0.01 to 1, x is in the range of 1 to 4, y is in the range of 0.01-1, wherein the catalyst has a total sulphur and/or phosphorus content of 1-10 wt.%, a surface are of 50-200 m²/g, a pore volume of at least 0.2 ml/g, and an average pore diameter of at least 50 Å.

The invention also pertains to a process for manufacturing the catalyst and to its use in esterification processes.

## Description

The present invention pertains to a solid acid catalyst, a process for its manufacture, and its use, in particular in the manufacture of biodiesel.

Biodiesel is a sustainable and renewable fuel produced from various oils, fats and free fatty acids. Conventional feedstocks include feedstocks of vegetable and animal lipid materials, more specifically frying and cooking fats (henceforth referred to as "waste fats"), industrial greases, vegetable non-edible oils, tall oil resulting from kraft pulping.

In the first step, these materials are reacted with alkyl alcohols. The resulting product is a mixture of fatty acid esters whose application properties as fuel is similar to the conventional petrodiesel fuel obtained from petroleum. Today for the most part the biodiesel is manufactured by the transesterification reaction of glycerides with mono-alcohols. In this reaction, one molecule of triglyceride reacts with one, two, or three molecules of mono-alcohol (most commonly methanol), producing one, two, or three molecules of mono-alkyl fatty esters and water, respectively. Current biodiesel processes typically use an excess of alcohol to shift the chemical equilibrium, increase the rate of the forward reaction, and help fat solubilisation in the liquid mixture. The transesterification is typically catalysed by a homogeneous base catalyst. NaOH, KOH and their corresponding alkoxides are the most used, as described by B. Gutsche in Fett/Lipid 99 (12), 418 (1997). The manufacturing of biodiesel employing heterogeneous base catalysts has been successfully implemented as described in EP-A-1 352 893, US patent 5,908,946 and US patent 6,147,196. Note that using base catalysts requires a feedstock with low free fatty acid (FFA) content, typically below 1% FFA, but preferably below 0.5% FFA, as well as a low water content, typically below 500 ppm. The above conditions can be met only by some edible virgin oils. Conversely, waste oil and fat contain typically between 4% FFA and 20% FFA. Consequently, a pre-esterification step is necessary, which typically uses an acid catalyst. Thus, biodiesel manufacturing from waste oil or fat must employ two catalysts, an acid for the esterification pre-treatment, and a base for the transesterification stage. If homogeneous catalysts are employed, costly operations are necessary. These include chemical neutralisation, washing, separation of organic and aqueous phases, and recovery of the alcohol (typically methanol) from the waste water streams by distillation. These additional separation steps are cumbersome and costly. Therefore, there is a strong incentive for using heterogeneous catalysts for biodiesel production.

Another route to the above-mentioned mono-alkyl esters is the direct esterification of FFA with mono-alcohols. In this reaction, one molecule of FFA reacts with one molecule of alcohol, producing one molecule of mono-alkyl fatty ester and one molecule of water. This esterification is catalysed by strong acid catalysts. Current biodiesel processes typically use an excess of alcohol to shift the chemical equilibrium and to increase the rate of the forward reaction. Fatty acid waste oils contain variable amounts of FFA, typically between 5% FFA and up to 100% FFA. These oils and fats are available from various sources, including waste frying oils from restaurants and industrial food processing, yellow grease and animal fats, as well as the tall oil obtained by paper milling. The prices of high-FFA oils are typically less than 50% than those of crop oils. Consequently, these are valuable sources for making low-cost biodiesel with the benefits of avoiding environment pollution

An alternative approach to tackle the variability of raw materials is the full hydrolysis of triglycerides to fatty acids, which can be further submitted to esterification, as described further in this invention. A suitable method is subcritical water hydrolysis that can take place at temperatures of 270 °C to 320 °C and pressures of 10 MPa to 20 MPa. The reaction time can be significantly reduced by an autocatalytic effect due to the initial FFA present in the feedstock [Minami, Saka, Fuels, 2479, 2006]. Up to 90% of the triglycerides can be converted to fatty acid in one step.

The Cetane Number (CN) is an important combustion characteristic of a diesel-type fuels. A minimum CN of 49 is required by the norm DIN 14214. It is known that the CN of fatty acid methyl esters (FAME) increases with the chain length, but decreases with the unsaturation in the hydrocarbon chain. The presence of fatty acid esters of long-chain alcohols, such as butanols or 2-ethyl-hexanol (2EH), increases the CN and as result the biodiesel's combustion capability, but does not affect its viscosity. For example 2EH fatty acid ester has a high CN (Kothe at al., Fuel 82, 971, 2003) and should improve also the cloud point (J. Am. Oil. Chem. Soc. 74, 951, 1997). Hence, the fatty esters of longer chain alkyl alcohol improve the quality of biodiesel mixtures originating from highly unsaturated feedstock, as rapeseed, palm, sunflower or soybean oils. Note that 2EH is a low cost byproduct of n-butyraldehyde production.

Typically, esterification is catalysed by strong acids, such as H₂SO₄, HCl, or p-Me(C₆H₄)SO₃H. A fundamental disadvantage of using as a homogeneous catalyst is that it must be neutralised, washed and removed from the product. These operations increase both the capital investment and the operational costs, require additional chemicals and generate contaminated water effluents that must submitted to costly treatment. The low legal limit of sulphur impurities allowed in diesel (currently < 20 ppm) also increases separation costs when sulphur-containing homogeneous catalysts such as H₂SO₄ or p-Me(C₆H₄)SO₃H are used.

Heterogeneous catalysts remain active in-situ for longer periods, do not require chemical neutralisation and do not generate harmful waste. These characteristics greatly simplify the technology. When the solid catalyst finally deactivates, it can be regenerated, recycled and reused. In general, solid base catalysts are preferred for transesterification and solid acid catalyst for esterification.

Solid acid catalysts (SAC) are suitable for catalysing fatty acid esterification. Acid resins, such as Nafion and Amberlyst, exhibit good activity, but can be employed only at low to moderate temperatures, in general bellow 130 °C. The use of niobic acid was described recently in US patent application 2007/0232817 A1. Metal oxides can be also applied with better results, since allow using higher reaction temperatures, up to 200°C, with higher productivity and better opportunity for heat integration (Kiss, Dimian and Rothenberg, Energy and Fuels, 2008, 22,598). The use of heterogeneous catalysts enables performing the reactions at higher temperatures, resulting also in better use of energy from the heat integration viewpoint.

Biodiesel manufacturing processes based on homogeneous catalysis require stainless steel corrosion resistant equipment, because of the handling of strong acid and base effluents. Conversely, a process that is based on a solid stationary catalyst, as proposed here, requires less and cheaper hardware.

The present invention pertains to a solid acid catalyst capable of carrying out the esterification of a feedstock containing 0.5%-100% free fatty acids with monoalkyl C1 to C8 alcohols for producing biodiesel.

The catalyst according to the invention is a solid acid catalyst of the formula M1ₐM2_{b}Oₓ(AO₃H)_{y}, wherein M1 and M2 may be the same or different and wherein M1 is selected from one or more of the transition metals, indium and tin and M2 is selected from one or more of the transition metals, lanthanide metals, indium and tin, A is selected from sulphur, phosphorus and mixtures thereof, a is in the range of 0.01 to 1, b is in the range of 0.01 to 1, x is in the range of 1 to 4, and y is in the range of 0.01-1, wherein the catalyst has a total sulphur and/or phosphorus content of 1-10 wt.%, a surface are of 50-200 m²/g, a pore volume of at least 0.2 ml/g, and an average pore diameter of at least 50 Å.

Within the context of the present specification the indication transition metals refers to the elements with atomic numbers 21-30, 39-48, and 71-80.

Within the context of the present specification the indication lanthanide metals refers to the elements with atomic numbers 57-71.

The catalyst according to the invention has large pores, highly acidic active sites and a tunable surface hydrophobicity. The surface hydrophobicity, pore size, pore volume, pore diameter, and number and strength and spread of the active sites are the key parameters. If the catalyst is too hydrophilic (or indeed has too many active sites), it will deactivate through water adsorption. On the other hand, if the surface is completely hydrophobic with very few active sites, the catalytic activity will be too low. By controlling the surface hydrophobicity and the number, strength and spread of active sites one can optimise the activity and stability parameters. In the present invention the surface hydrophobicity is tuned by the combination of the content of sulphur and/or phosphorus, the surface area, and the duration and temperature of the calcination step.

The catalyst according to the invention possesses high activity for biodiesel synthesis at temperatures between 100°C and 200°C, preferably between 120°C and 180°C. The catalyst is multi-substrate, exhibiting similar activity for fatty acids in the series C12 to C18 and for normal and branched alcohols in the series C1 to C8. The activity for methanol is the highest, and about twice the activity for 2-ethyl-hexanol in identical reaction conditions.

The invented catalyst shows practically 100% selectivity towards the formation of the alkyl ester. Despite the high acidity, no ethers or other by-products are detected, even when the alcohol excess is up to five times the stoichiometric amount, at temperatures between 120°C and 180°C.

Robustness tests showed that the invented catalyst keeps almost constant activity over longer period when the water concentration in liquid phase is kept low, preferable below 2 wt%. This low concentration can be achieved by carrying out the reaction at temperatures over 100 °C and at best in a reactive distillation environment. The catalyst keeps its integrity over long operation periods, providing that the reaction temperature is below 180 °C and the water content in the organic liquid phase is below 2 wt%.

In the catalyst according to the invention it preferred for M1 to selected from zirconium, titanium, yttrium, vanadium, niobium, molybdenum, tin, tungsten, and mixtures thereof. The use of zirconium, tin, titanium, and niobium is particularly preferred.

M2 is preferably selected from zirconium, titanium, yttrium, vanadium, niobium, molybdenum, tin, tungsten, or a lanthanide metal, or mixtures thereof. The use of zirconium, tin, titanium, and niobium is particularly preferred.

In a particularly preferred embodiment of the present invention M1 and M2 are the same, and are zirconium, tin, titanium, or niobium, more in particular zirconium.

In the catalyst according to the invention, A is sulphur and/or phosphorus. This means that the catalyst according to the invention has been subjected to a sulphonation treatment and/or a phosphonation treatment. It is preferred for the catalyst to be either a sulphonated catalyst or a phosphonated catalyst. In other words, it is preferred for A to be S or P.

The catalyst according to the invention has a surface area (nitrogen adsorption; BET) of 50-200 m²/g. In more specific embodiments, the catalyst has a surface area of 60-120 m²/g, in particular of 70-100 m²/g.

The catalyst according to the present invention has an average pore diameter (nitrogen adorption; BET) of at least 50 Å. If the average pore diameter is below this value, the pores are to small to allow the reactants to access the active sites. More in particular, the catalyst has an average pore diameter of at least 70 Å, in particular between 70 and 150 Å.

The catalyst according to the invention has a pore volume which is at least 0.2 ml/g. More in particular, the catalyst has a pore volume which is at least 0.22 ml/g, in particular between 0.22 and 0.4 ml/g.

The catalyst has a total sulphur and/or phosphorus content of 1-10 wt.%. In a preferred embodiment, the catalyst has a total sulphur and/or phosphorus content of 3-10 wt.%, more preferably 6-10 wt.%.

The present invention also pertains to a process for preparing a particulate solid acid catalyst. The process according to the invention comprises the steps of
- melting metal salt precursors at a pressure of 0.1 up to 1 bar to form a melt,
- reducing the pressure to remove volatile components and form a solid catalyst,
- subjecting the resulting material to a calcination step in the presence of oxygen at a temperature of 400-700°C for a period of 1-20 hours
- subjecting the catalyst to a sulphonation and/or phosphonation step by contacting it with a sulphonation and/or phosphonation agent
- subjecting the sulphonated and/or phosphonated catalyst to a calcination step in the presence of oxygen at a temperature of 400-700°C for a period of 1-20 hours

In the first step of the process according to the invention, metal salt precursors are melted. Suitable metal salt precursors include nitrates, chlorides, and ammonium compounds. For the nature of the metals reference is made to what is stated above for M1 and M2.

The melting takes place at a pressure of 0.1-1 bar. While operating at a value of 1 bar or higher is possible in principle, it is preferred for the melting step to take place under reduced pressure, e.g., at a pressure of 0.1-0.9 bar, more in particular at a pressure of 0.2-0.8 bar.

The next step in the process of the invention is reducing the pressure. This results in removal of the volatile components, and formation of a solid catalyst The pressure applied in this step is generally at least 0.1 bar lower than the pressure applied during the melting step.

In the next step, the material is subjected to a calcination step in the presence of oxygen. The calcination is carried out at a temperature of 400-700°C, for a period of 1-20 hours. The purpose of the calcination is to convert the metal salts in the solid material into the corresponding metal oxides. The exact calcination time and temperature are interdependent, and it is within the scope of the person skilled in the art to optimise calcination time and temperature. If the calcination is too severe, the pore structure of the catalyst may be destroyed. Conversely, if the calcination is too mild, the salt precursor groups, like nitrate, chloride, and ammonium, may not be removed from the surface. The calcination should be carried out under such conditions that at least 95% of the metal salts is converted into the corresponding metal oxide. In one embodiment substantially all of the metal salts are converted into their corresponding oxides. The combination of a calcination temperature in the range of 500-650°C with a calcination time of for four to eight hours is a particular embodiment of the present invention. The oxygen-containing gas used in the calcinations step is generally air, although the use of other oxygen-containing gases is also envisaged.

The calcined catalyst is subsequently subjected to a sulphonation and/or a phosphonation step, wherein the material is contacted with appropriate sulphonation and/or phosponation agent. As indicated above, while it is possible to perform both a sulphonation step and a phosphonation step it is preferred to carry out either a sulphonation step or a phosphonation step. If both a sulphonation step and a phosphonation step are carried out, they can be carried out sequentially, or simultaneously. Suitable sulphonation agents include H₂SO₄, HClSO₃, and similar sulphur-containing materials. In a preferred embodiment, chlorosulphonic acid HClSO₃ is used. Suitable phosphonation agents include H₃PO₄, H₂PO₃, H₂ClPO₃, and similar phosphorus-containing materials. In a preferred embodiment H₃PO₄ is used. While the present invention encompasses both sulphonated and phosphonated catalysts, sulphonated catalysts are presently considered preferred. Suitable sulphonation and phosphonation processes are known in the art and require no further elucidation here.

The sulphonated and/or phosphonated product is then recovered, dried if necessary, e.g., for 10 to 20 hours, and recalcined in the presence of oxygen at a temperature of 400-700°C for a period of 1-20 hours. Again, the exact calcination time and temperature are interdependent, and it is within the scope of the person skilled in the art to optimise calcination time and temperature. If the calcination is too severe, the pore structure of the catalyst may be affected.

The combination of a calcination temperature in the range of 500-650°C with a calcination time of for four to eight hours is a particular embodiment of the present invention. The oxygen-containing gas used in the calcinations step is generally air, although the use of other oxygen-containing gases is also envisaged.

The catalyst according to the invention is suitable for reactions involving large molecules, such as the esterification of fatty acids with mono alcohols.

The particle size of the catalyst will depend on the process in which it is used. In one embodiment, the catalyst is shaped to form particles with a diameter of 0.1-10 mm. Where the catalyst is to be used in reactive distillation, it may be attractive to shape the catalysts into particles with a diameter of 0.5-1.5 mm, more in particular 0.8-1 mm. Suitable shaping techniques are known in the art and encompass pelletising and other shaping processes. These particles have a size that makes them particularly suitable for use in structured packing. For used in other liquid-phase reactors the catalyst can be shaped in larger pellets, preferably 2-6 mm in diameter.

The catalyst according to the invention has been found to be very selective, in particular as regards the formation of byproducts in esterification processes where a large amount of alcohol is present.

The present invention therefore also pertains to a process for the esterification of carboxylic acids, wherein a carboxylic acid is reacted with an alcohol in the presence of a catalyst as described above to form an ester.

The carboxylic acid may be any carboxylic acid, or a mixture of two or more different carboxylic acids. In one embodiment, the carboxylic acid or acids have 10-18 carbon atoms.

The alcohol may be any alcohol. In one embodiment the alcohol has 1-15 carbon atoms.

The reaction is generally carried out at a temperature between 100 and 220°C, in particular between 150 and 200°C. As is known in the art, it is preferred for the esterification reaction to be carried out while simultaneously removing the water formed in the reaction.

In one embodiment of the present invention a feedstock is used which comprises C₁₀-C₁₈ fatty acids in combination with fatty acid triglycerides. The amount of fatty acid and triglycerides in the feed varies with the source. In general, the feedstock contains 1-100 wt.% of fatty acids. In one embodiment, where the feedstock is derived from oil and non-waste fats, the feedstock contains between 1-30 wt.% of C₁₀-C₁₈ fatty acids and 99-70 wt.% of triglycerides of C₁₀-C₁₈ fatty acids. In another embodiment, where the feed is derived from waste fats, the feed generally contains 50-100% of C₁₀-C₁₈ fatty acids and 0-50 wt.% of triglycerides of C₁₀-C₁₈ fatty acids.

The present invention also pertains to a process in which the esterification of carboxylic acids using the catalyst according to the invention is carried out in a reactive distillation unit. In this embodiment, the present invention pertains to a process for the manufacture of carboxylic acid esters through reactive distillation, wherein a carboxylic acid is reacted in a reaction section of a reactive distillation column with an alcohol under esterification conditions in the presence of a catalyst as described above to form an ester, wherein a first supply stream comprising the carboxylic acid and a second supply stream comprising an alcohol are supplied to the reactive distillation column, wherein the first supply stream is supplied to the column at a first entry level located just above or at the top of the reaction section and the second supply stream is supplied to the column at a second entry level located in or just below the reaction section and below the first entry level, and wherein a bottom stream comprising the ester formed is obtained and a top stream comprising unreacted first alcohol and water.

In one embodiment, the unit is provided with a third supply stream comprising an entrainer. Where an entrainer is used it is preferred for the supply stream containing the entrainer to be supplied to the column at a third entry level located above the first entry level. In this embodiment, the top steam comprising unreacted first alcohol and water may also comprise residual entrainer.

In one embodiment, the entrainer is a hydrocarbon with 1-20 carbon atoms. Suitable entrainers encompass hydrocarbons boiling in the gasoline range, e.g. C₅-C₁₂ hydrocarbons.

In one embodiment, the entrainer comprises a second alcohol, the second alcohol having a higher molecular weight than the alcohol which is used as reactant. In this embodiment the second alcohol has the combined function of entrainer of water and co-reactant in the esterification and transesterification reactions. In this embodiment, it is preferred for the reactant alcohol, further also indicated as the first alcohol, to be selected from methanol, ethanol, n-propanol, and mixtures thereof, and for the entrainer alcohol to be is selected from mono-alkylalcohols with 5 to 10 carbon atoms, in particular 6 to 8 carbon atoms. Where the entrainer is a second alcohol, it is preferred for the supply stream containing the entrainer to be supplied to the column at a third entry level located above the first entry level. In this embodiment, the top steam comprising unreacted first alcohol and water may also comprise residual alcohol entrainer.

The present invention is elucidated by the following examples, without being limited thereto or thereby.

### Example 1 Catalyst synthesis

Appropriate metal nitrate precursors were weighed into a crucible and mixed with a spatula. A maximum of six crucibles were placed into a vacuum oven set at 115°C. The precursors were allowed to melt for about five minutes, after which the pressure was lowered to <10 mbar in about 15 min. The latter step was performed carefully to prevent vigorous boiling. After four hours, the precursors were placed in a muffle oven and calcined for four to eight hours at 500-650 °C in static air (ramp rate: 300 °C/h, exact calcination temperature and duration depending on composition of mixed oxide). The resulting solid was pulverized, ground, and sieved in fractions of 125-212 micrometer (selectivity assessment) and <125 micrometer (TPR, TGA, XRD, and BET measurements). The final metal concentration was calculated from the amount of precursor weighed (corrected for the water content by means of ICP-MS (Inductive Coupled Plasma Mass Spectrometry)). The next step is the sulphonation. Then the solid is recovered and dried for 15 hours at 110 °C, and calcined for four to eight hours at 500-650 °C in static air (ramp rate: 300 °C/h, exact calcination temperature and duration depending on composition of mixed oxide). As a reference catalyst, a sample of sulphated zirconia was synthesised, in which 15 ml sol. H2SO4 1 N / g Zr(OH)₄ was used as a sulphonation agent (this reference catalyst is called hereafter YD1). The new catalyst is called here YD2.

Table 1 presents the key catalyst characterisation parameters. The results show that the pore diameter and the pore volume are significant larger for the new YD2 catalyst. In addition the pore size distribution is broader at larger sizes for the first, while for the second is very narrow around the mean at low sizes.

Another test of significance is the total acidity, measured by TPD analysis. It can be observed again a relevant difference. YD1 shows that most of the acid sites are located at low temperatures (150, 220 °C) as well as around 400°C. In contrast, YD2 shows a large peak placed at higher temperature, namely at 530-540 °C. These sites over 400°C can be characterised as "super-acidic". These two analyses demonstrate that YD2 has larger pores than the reference catalyst YD1, and exhibits more super-acidic sites. Its sulphur content is also higher.

**Table 1 Catalyst characterization by surface and pore size**

| Catalyst | Surface area [m²g⁻¹] | Pore volume [cm³g⁻¹] | Pore diameter mean / max [nm] | Sulphur content [%] |
|---|---|---|---|---|
| YD1 comparative | 94 | 0.198 | 7.5 /7.8 | 3.4 |
| YD2 | 85 | 0.300 | 9.4/16.5 | 7.2 |

### EXAMPLE 2 Catalyst activity for biodiesel manufacturing

An amount of 0.1 mol oleic acid is charged into an autoclave together with an amount of catalyst YD2 of 2.5% with respect to the fatty acid. After heating the reaction mixture at the temperature of 140 °C and flushing with nitrogen, the corresponding amount of preheated alcohol is added. The reaction takes place autogenously at the pressure developed by the vapour-liquid equilibrium. The table below shows the yield expressed as the mol of ester per mol of acid versus time for different molar ratios R methanol/fatty acid. The reaction rate is exceptionally high and suitable for a reactive distillation set-up. The initial reaction rate is of about 6 percent fatty acid conversion per minutes. About 90% from the equilibrium conversion can be achieved in less than 30 minutes. At methanol/oil ratio R=2 the equilibrium conversion reaches 85%, while at a ratio R=5 it may reach over 95%.

**Table 2 Activity of catalyst YD2 in the esterification of oleic acid with methanol at 140 °C**

| Time (min) | 10 | 20 | 30 | 60 | 90 | 120 |
|---|---|---|---|---|---|---|
| R 1:1 | 0.542 | 0.594 | 0.613 | 0.629 | 0.645 | 0.668 |
| R 2:1 | 0.625 | 0.771 | 0.824 | 0.846 | 0.854 | 0.852 |
| R 5:1 | 0.645 | 0.805 | 0.872 | 0.940 | 0.956 | 0.960 |

### EXAMPLE 3 Effect of temperature

The experimental setup is as in Example 2 excepting the reaction temperature. Rising the temperature can increase significantly the reaction rate while affecting only slightly the equilibrium conversion, as it can be seen from Table 3.

**Table 3 Effect of temperature on the activity of catalyst YD2.**

| Time (min) | 5 | 10 | 20 | 30 | 60 | 120 |
|---|---|---|---|---|---|---|
| T=130 °C | 0.345 | 0.518 | 0.563 | 0.603 | 0.624 | 0.668 |
| T=140 °C | 0.457 | 0.542 | 0.594 | 0.613 | 0.645 | 0.682 |
| T=150 °C | 0.561 | 0.645 | 0.675 | 0.682 | 0.692 | 0.705 |

### EXAMPLE 4 Effect of catalyst amount on the reaction rate

The experimental setup is as in Example 2 but now the amount of catalyst is varied from 2.5 wt% with respect to fatty acid to 5% and 10%. Results are given in Table 4. The reaction rate can be increased by employing more catalyst, as it can be seen from Table 4.

**Table 4 Effect of the amount of the catalyst on the reaction rate**

| Time (min) | 5 | 10 | 15 | 20 | 30 |
|---|---|---|---|---|---|
| 2.50% | 0.345 | 0.518 | 0.542 | 0.563 | 0.603 |
| 5% | 0.465 | 0.56 | 0.597 | 0.625 | 0.66 |
| 10% | 0.506 | 0.59 | 0.622 | 0.645 | 0.68 |

### EXAMPLE 5 Kinetics for 2-ethyl-hexanol

The reaction conditions are the same as in Example 2, except the fact that the methanol was replaced by 2-Ethyl hexanol. Table 5 shows the results. The reaction rate is still good, but sensible lower as before. The equilibrium conversions are higher as with methanol. At a molar ratio of two it can reach over 98%.

**Table 5 Catalyst activity for 2-ethyl-hexanol**

| Time (min) | 10 | 20 | 30 | 60 | 90 | 120 |
|---|---|---|---|---|---|---|
| R 1:1 | 0.089 | 0.235 | 0.364 | 0.617 | 0.73 | 0.784 |
| R 2:1 | 0.117 | 0.305 | 0.486 | 0.841 | 0.96 | 0.985 |

### EXAMPLE 6 The relative activity of catalysts

The experimental conditions are as in Example 3, with the exception that the molar ratio of reactants was 1:1, the amount of catalyst was 6% with respect to the oil and reaction temperature of 160 C. The table below shows the results as yield in fatty ester versus time for the catalysts YD1 and YD2. It can be seen that the activity of YD2 is much higher than of YD1. The initial reaction rate is at least twice higher.

**Table 6 Comparison of activity of YD1 and YD2 catalysts**

| Time (min) | 10 | 20 | 30 | 60 | 90 | 120 |
|---|---|---|---|---|---|---|
| YD1 | 0.125 | 0.381 | 0.615 | 0.78 | 0.844 | 0.86 |
| YD2 | 0.263 | 0.682 | 0.843 | 0.885 | 0.89 | 0.88 |

### EXAMPLE 7 Testing for leaching of sulphonic groups

In a first approach, the presence of sulphate ions in solution was checked with BaCl2 and KOH titration. For example, after a typical run at 130 °C and quantitative catalyst recovery by filtration, the resulting mixture was washed with water for the extraction of sulphate ions. No sulphate ions were found. For comparison, when commercial sulphated zirconia was mixed with water at 25 °C, the same test showed clearly the leaching of sulphate ions. A separate series of experiments, where the catalyst YD2 was filtered after 30 min of reaction and fresh catalyst was added after a further 45 min, confirmed that sulphonic groups do not leach from the catalyst into solution under the reaction conditions.

### EXAMPLE 8 Robustness and reproducibility

Experimental set-up is as in Example 4 excepting that the initial catalyst amount was 10 wt% with respect to oleic acid. After reaction for 2 hours, the catalyst was quantitatively recovered by filtering on very fine paper and dried at 120 °C for one hour. In the next run the amount of reactants was adapted to respect 10% weight catalyst ratio. After four complete runs over three hours the activity remains practically unchanged. The equilibrium conversion of about 90 % is reached in about 30 minutes.

## Claims

1. A solid acid catalyst of the formula M1ₐM2_{b}Oₓ(AO3H)_{y}, wherein M1 and M2 may be the same or different and wherein M1 is selected from one or more of the transition metals, indium and tin and M2 is selected from one or more of the transition metals, the lanthanide metals, indium, and tin, A is selected from sulphur, phosphorus, and mixtures thereof, a is in the range of 0.01 to 1, b is in the range of 0.01 to 1, x is in the range of 1 to 4, y is in the range of 0.01-1, wherein the catalyst has a total content of sulphur and phosphorus of 1-10 wt.%, a surface are of 50-200 m²/g, a pore volume of at least 0.2 ml/g, and an average pore diameter of at least 50 Å.

2. Catalyst according to claim 1, wherein M1 is selected from zirconium, titanium, yttrium, tin, vanadium, niobium, molybdenum, tungsten, and mixtures thereof.

3. Catalyst according to claim 1 or 2, wherein M2 is selected from zirconium, titanium, yttrium, tin, vanadium, niobium, molybdenum, tungsten, lanthanide metals, and mixtures thereof.

4. Catalyst according to claim 2 or 3, wherein M1 and M2 are the same, and are zirconium, tin, titanium, or niobium.

5. Catalyst according to any one of the preceding claims wherein the catalyst has a surface area of 60-120 m²/g, in particular of 70-100 m²/g.

6. Catalyst according to any one of the preceding claims wherein the average pore diameter is at least 70 Å, in particular between 70 and 150 Å.

7. Catalyst according to any one of the preceding claims wherein the catalyst has a pore volume which is at least 0.22 ml/g, in particular between 0.22 and 0.4 ml/g.

8. Method for manufacturing a particulate solid acid catalyst, comprising the steps of
• melting metal salt precursors at a pressure of 0.1 up to 1 bar to form a melt,
• reducing the pressure to remove volatile materials and form a solid product,
• subjecting the resulting material to a calcination step in the presence of oxygen at a temperature of 400-700°C for a period of 1-20 hours
• subjecting the catalyst to a sulphonation and/or phosphonation step by contacting it with a sulphonation and/or phosphonation agent
• subjecting the sulphonated and/or phosphonated catalyst to a calcination step in the presence of oxygen at a temperature of 400-700°C for a period of 1-20 hours.

9. Method according to claim 8, wherein the metal salt precursors are selected from metal nitrates, chlorides, and ammonium compounds.

10. Process for the esterification of carboxylic acids, in particular of mixtures of carboxylic acids, wherein the carboxylic acid is reacted with an alcohol in the presence of a catalyst according to any one of claims 1-7.

11. Process according to claim 11, wherein the carboxylic acid is in a feedstock which comprises C₁₀-C₁₈ fatty acids, optionally in combination with fatty acid triglycerides.

12. Process according to claim 10 or 11 wherein the alcohol is a C₁-C₁₅ alcohol.

13. Process according to any one of claims 10-12 which is carried out in a reactive distillation column wherein a carboxylic acid is reacted in a reaction section of a reactive distillation column with an alcohol under esterification conditions in the presence of a catalyst as described above to form an ester, wherein a first supply stream comprising the carboxylic acid and a second supply stream comprising an alcohol are supplied to the reactive distillation column, wherein the first supply stream is supplied to the column at a first entry level located just above or at the top of the reaction section and the second supply stream is supplied to the column at a second entry level located in or just below the reaction section and below the first entry level, and wherein a bottom stream comprising the ester formed is obtained and a top stream comprising unreacted first alcohol and water.

14. Process according to claim 13 wherein the unit is provided with a third supply stream comprising an entrainer, the third supply stream being supplied to the column at a third entry level located above the first entry level.

15. Use of a catalyst according to any one of claims 1-7 in the manufacture of esters from alcohols and carboxylic acids.
